# EUROPEAN PATENT APPLICATION

(11) **EP 0 714 884 A1**
(43) Date of publication of application: **05.06.1996**
(21) Application number: 95118778.0
(22) Date of filing: 29.11.1995
(51) Int. Cl.: C07C 227/40, C07C 277/00, C07C 277/08, C12P 13/04

(54) **A method for the production of a basic amino acid**

(30) Priority: 30.11.1994 JP 296072/94
(71) Applicant: Ajinomoto Co., Inc., Tokyo 104 (JP)
(72) Inventor: Kaneko, Toyokazu, c/o Kawasaki Plant, Kawasaki-shi, Kanagawa-ken (JP); Hisamitsu, Kunio, c/o Kawasaki Plant, Kawasaki-shi, Kanagawa-ken (JP)
(74) Representative: Strehl Schübel-Hopf Groening & Partner

(57) **Abstract**

This method results in a purified basic amino acid which does not cause problems in filterability even when a neutral or acidic aqueous solution containing the basic amino acid is processed by filtration.
A method for the purification of a basic amino acid wherein an alkaline aqueous solution of the amino acid produced by a fermentation method is filtered through a tight-ultrafiltaration membrane.

## Description

### [Field of the invention]

The present invention relates to a method for the purification of a basic amino acid which is produced by fermentation methods and contains protein-based impurities.

### [Prior art]

Basic amino acids such as L-arginine are produced by fermentation methods. In one of the methods to purify such amino acids, a fermentation broth, after removal of cells or directly as it is, is loaded onto an ion-exchange resin to adsorb the basic amino acid, and then subjected to elution, followed by crystallization directly or via decolorization with activated carbon. The fermentation broth contains various impurities, including water-soluble proteins. The water-soluble proteins are adsorbed on the ion-exchange resin and then eluted with an alkaline eluent together with the basic amino acids. They precipitate out in the subsequent crystallization step, thus making the impurities difficult to remove. Accordingly, a conventional removal method employs a procedure in which a protein coagulant is added to the aqueous solution of the amino acid and the mixture is heated to coagulate the proteins which are then filtered off.

### [Problems to be solved by the invention]

The basic amino acids are employed mainly as starting materials for pharmaceuticals such as nutrition formulations including amino acid infusion solutions or neutralizing agents, and are usually used at a pH of from neutrality to weak acidity. However, when a basic amino acid is dissolved to form an aqueous solution while adjusting it between neutral and weakly acidic and then sterilized by filtering through a membrane filter, the filtration rate is substantially reduced.

The membrane filter means herein a micro-filtration membrane used for sterilization which has a pore size of from 0.22 to 1.0µm.

An objective of the present invention is to provide a method for the purification of a basic amino acid which does not cause problems of filterability even when a neutral or weak acidic aqueous solution containing the basic amino acid is treated by filtration through a membrane filter.

### [Means to solve the problems]

After making much effort to solve the problems mentioned above, we found that, since the basic amino acid solution is alkaline, the removal of the impurities such as proteins using an ordinary purification method can not be conducted sufficiently and the impurities thus remaining undergo precipitation at an acidic pH, resulting in the inhibition of filtration. Thus, since most of the amino acid products are in the forms of the salts not only in the cases of neutral and acidic amino acids but also in the case of basic amino acids, their aqueous solutions are subjected to filtration at a pH of from neutrality to weak acidity for purificatin. In a neutral or weak acidic aqueous solution, colloidal substances such as proteins tend to coagulate, thereby facilitating the removal by filtration. However, in the case of basic amino acids which are not salt form, the aqueous solutions are alkaline and the colloidal substances are poorly coagulated and occasionally pass through the filter. In addition, the alkaline condition causes a higher solubility of the proteins, some of which are present in dissolved form. Such behavior of the proteins and other impurities was now found to cause difficulties in filtration of the alkaline aqueous solution of the basic amino acid as mentioned above.

Therefore, for the purpose of improving the filtration at a pH between neutral and weakly acidic, it is necessary to remove completely the substances which behave as mentioned above and to remove the substances which are dissolved under alkaline conditions and precipitate under acidic conditions.

One of the methods of removing such substances is neutralization of the alkaline aqueous solution of a basic amino acid with an acid such as hydrochloric acid followed by the filtration of the neutralized solution through a membrane filter. However, this method is not suitable as a means of obtaining an alkaline aqueous solution or crystals of the basic amino acid since it requires an acid for the neutralization and a treatment with the ion exchange resin for removing anionic components such as chlorine ions.

Accordingly, we attempted to use an ultrafiltration membrane (pore size of from 0.002 to 0.1µm), which is usually employed to remove the pyrogens in pharmaceutical amino acids used as the starting materials of the infusion fluids and which has a smaller pore size than membrane filters for micro-filtration, in the purification step prior to the crystallization step for the purpose of the removal of the impurities mentioned above. However, in spite of the use of the ultrafiltration membrane having a pore size far smaller than that of membrane filters, the filterability of the aqueous solution in which the basic amino acid obtained was dissolved and which was adjusted to a pH of from neutrality to weak acidity was not improved significantly.

Then, we further studied to develop highly efficient filtration methods and finally found that a filtration method using a recently commercially available tight-ultrafiltration membrane is optimal for this purpose, thereby establishing the present invention.

Accordingly, the present invention relates to a method for the purification of a basic amino acid wherein an alkaline aqueous solution of the amino acid produced by a fermentation method is filtered through a tight-ultrafiltration membrane.

The basic amino acids applicable in the method of the present invention are not specifically limited, and include L-lysine, L-arginine, L-ornithine, L-histidine and the like. The method according to the present invention is most effective in the case where the aqueous solution is highly alkaline and can be purified by crystallization, and, from this point of view, L-arginine, L-lysine and L-ornithine are preferable, with L-arginine being particularly preferable.

The alkaline aqueous solution of the basic amino acid is preferably in a somewhat purified physical and chemical state, since those containing a large amount of insoluble substances such as cells, for example, a fermentation broth, are not filtered effectively. Examples of those which are preferable are an eluate obtained by the adsorption of the basic amino acid in the fermentation broth to a cation exchange resin followed by the removal of the non-adsorbed solution and followed by the elution of the basic amino acid with an aqueous solution of a base such as sodium hydroxide, a decolorized solution obtained from this eluate by decolorization with activated carbon, an aqueous solution in which crude crystals obtained by the crystallization of the basic amino acid from this eluate mentioned above have been dissolved, a decolorized solution obtained from this aqueous solution of the crude crystals by decolorization with activated carbon, and a decolorized solution obtained from this aqueous solution of the crude crystals by the addition of a protein coagulant followed by decolorization with activated carbon. The pH of the alkaline aqueous solution of the basic amino acid is close to the isoelectric pH, usually within the range of the isoelectric pH±0.5. The concentration of the basic amino acid in the aqueous solution is preferably below the saturation solubility of the amino acid at the temperature at which the filtration is conducted and which is established based on the prevention of the thermal decomposition of the basic amino acid and the tolerable temperature of the tight-ultrafiltration membrane. For easier operation, a concentration close to that of the saturation solubility is preferable. For example, when an aqueous solution of L-arginine is filtered through a tight-ultrafiltration membrane at 40°C, the concentration of L-arginine is preferably from 20 to 30 g/dl since the saturation solubility of L-arginine at 40°C is about 35 g/dl.

The tight-ultrafiltration membrane is a membrane which is considered to be an intermediate between an ultrafiltration membrane and a reverse-osmosis membrane with regard to the fractionation molecular weights and which has a fractionation molecular weight within the range of from a few hundreds to a few thousands. The NaCl rejection ratio is used frequently to represent the fractionation performance of a membrane. The tight-ultrafiltration membrane employed in the present invention has a NaCl rejection ratio of 1 to 50 %, preferably 5 to 30 %. The material of the membrane may be chosen from acetylcellulose-based, polyamide-based, polybenzoimidazole-based or polysulfone-based materials.

With regard to the filtration conditions, the temperature is 5 to 95°C, preferably 20 to 60°C. Although the pressure may be at any level below the tolerable pressure limit of the particular tight-ultrafiltration membrane employed, it is preferably selected considering the efficiency since a much lower pressure prolongs the filtration period. Usually a pressure of from 5 x 10⁵ to 100 x 10⁵ Pa (5 to 100 kg/cm²) is employed. As a filtration device, any commercially available device can be employed as it is.

By filtering an alkaline aqueous solution of a basic amino acid which was produced by a fermentation method and which contains protein impurities through a tight-ultrafiltration membrane, colloid particles and watersoluble proteins whose molecular weight in an aqueous solution is greater than the fractionation molecular weight do not pass through the filter, while the basic amino acid passes, thereby effecting the separation.

### Example 1

According to the method described in the examples in Laid-Open Japanese Patent Application Number 02-186995(1990), Brevibacterium flavum AJ12429 (FERM BP-2227) was cultured and 25 Kℓ of L-arginine fermentation fluid (L-arginine content: 750 kg) was obtained. This fermentation broth was adjusted to a pH of from 6.5 to 7 with hydrochloric acid, and then the cells were separated by centrifugation. Then the cell-free solution was loaded onto a cation-exchange resin (MITSUBISHI CHEMICAL CO., Diaion SK-1B, resin bed: 6Kℓ) to adsorb L-arginine, and then washed with 8Kℓ of water. Subsequently, 1N aqueous solution of NaOH was loaded to elute L-arginine. The eluate was concentrated under a reduced pressure to a volume of 1Kℓ, and then cooled to 10°C, thereby crystallizing L-arginine dihydrate. The crystals thus obtained were separated by centrifugation to yield 600 kg of crude crystals (L-arginine dihydrate, L-arginine content: 480 kg). 600 kg of the crude crystals were dissolved in purified water to attain a volume of 2500 ℓ at 60°C. The solution thus obtained was admixed with 0.5 ℓ of 10% aqueous solution of a protein coagulant, benzalkonium chloride(Kao SANYZOL), and the mixture was stirred for 1 hour. After addition of 6 kg of activated carbon, the mixture was stirred for further 45 minutes. Then, the decolorized solution was filtered through a ceramic filter precoated with Celite, and further 500 ℓ of purified water of 60°C were added and the solution was successively filtered to recover the residue in the filter. The volume of the decolorized solution thus obtained was 3,000 ℓ.

This decolorized solution was purified by a conventional method, a ultrafiltration method (UF method) as a comparative example, and the method of the present invention (L-RO method) to obtain the respective purified crystalline products.

### [Conventional method]

1,000 ℓ of the decolorized solution was filtered through a 0.5 µm membrane filter and then concentrated under reduced pressure (680 mmHg) to a volume of 300 ℓ, thereby effecting crystallization. The concentrated slurry was transferred to a crystallization vessel, which was cooled to 10°C while stirring and then obtained crystals were separated. The separated crystals were washed with a small amount of pyrogen-free water.

The wet purified crystals obtained amounted to 170 kg.

Then the wet purified crystals were dried under reduced pressure (720 mmHg) with a conical dryer while elevating the temperature stepwise from 65 to 95°C over a period of 12 hours.

The dried purified crystals were obtained in an amount of 136 kg.

### [UF method]

1,000 ℓ of the decolorized solution were filtered through an ultrafiltration membrane (NTU-3250, NITTO DEKO CO., fractionation molecular weight (as protein): approximately 6,000) under the conditions shown below.

| (UF conditions) | |
|---|---|
| Pressure | 2 x 10⁵ Pa (2 kg/cm²) |
| Flow rate | 10 ℓ/min |
| Temperature | 25 to 30°C |

The filtration of the decolorized solution was continued until the volume of the concentrated solution (processed solution) became 2.5 ℓ, and thereafter diafiltration with 10 ℓ of purified water was conducted to recover the remaining L-arginine. The volume of the permeate was 1,010 ℓ.

This permeate was subjected to a process similar to that for the filtrate through the 0.5 µm membrane filter in the conventional method, to obtain dried crystals.

The dried purified crystals were obtained in an amount of 135 kg.

### [L-RO method (the method of the present invention)]

1,000 ℓ of the decolorized solution were filtered through a tight-ultrafiltration membrane made from synthetic resin charged with sulfonyl groups (NTR-7410, NITTO DENKO CO., NaCl rejection ratio: 10%) under the conditions shown below.

| (L-RO conditions) | |
|---|---|
| Pressure | 10 x 10⁵ Pa (10 kg/cm²) |
| Flow rate | 10 ℓ/min |
| Temperature | 25 to 30°C |

The filtration of the decolorized solution was continued until the volume of the concentrated solution (processed solution) became 2.5 ℓ, and thereafter a diafiltration with 10 ℓ of purified water was conducted to recover the remaining L-arginine. The volume of the permeate was 1,010 ℓ.

This permeate was subjected to a process similar to that for the filtrate from the 0.5µm membrane filter in the conventional method, obtaining dried crystals.

The dried purified crystals were obtained in an amount of 136 kg.

The dried purified crystals were obtained by the three method described above were filtered under the condition shown below to compare the filterability. The pH was adjusted with 2N aqueous solution of HCl, and the filterability was evaluated by determining the time period required for the filtration. The results are shown in Table 1.

| (Conditions of filterability evaluation) | |
|---|---|
| Pressure | 2 x 10⁵ Pa (2 kg/cm²) |
| Concentration of L-arginine | 5g/dl |
| Temperature | 30°C |
| Volume to filter | 1.4 ℓ |
| Membrane filter | Millipore 0.22 µmGS |
| pH | 11, 7, 5, 3 |

**Table 1**

| Filtration period(second) of aqueous solution at each pH | | | | |
|---|---|---|---|---|
| Solution of purified crystals | pH 11 | pH 7 | pH 5 | pH 3 |
| Conventional method | 1010 | 1510 | 8000 | Occlusion |
| UF method | 850 | 1400 | 2640 | Occlusion |
| LR-O method | 930 | 910 | 880 | 920 |

The purified crystals obtained by the conventional method and UF method exhibited extremely poor filterability at a neutral to an acidic pH, especially at pH 3. On the other hand, the purified crystals obtained by L-RO method (the method of the present invention) exhibited no deterioration of the filterability in the range of a neutral to an acidic pH.

In addition to the improvement of the filterability, the L-RO process provided further improvement in removal of pyrogens.

### Example 2

1,000 ℓ of the decolorized solution obtained by a process similar to that in Example 1 were subjected to the L-RO method using a non-charged tight-ultrafiltration membrane (HR-20, Toray, NaCl rejection ratio: 15%) under the conditions shown below.

| (L-RO conditions) | |
|---|---|
| Pressure | 25 x 10⁵ Pa (25 kg/cm²) |
| Flow rate | 12 ℓ/min |
| Temperature | 20 to 25°C |

The filtration of the decolorized solution was continued until the volume of the concentrated solution (processed solution) became 2.5 ℓ, and thereafter diafiltration with 10 ℓ of purified water was conducted to recover the remaining L-arginine. The volume of the permeate was 1,020 ℓ.

This permeate was subjected to a process similar to that for the filtrate through the 0.5µm membrane filter in the conventional method, to obtain dried crystals.

The dried purified crystals were obtained at an amount of 132 kg.

The dried purified crystals obtained by the method described above were filtered under conditions similar to those in Example 1 to evaluate the filterability.

The results are shown in Table 2.

**Table 2**

| Filtration period (second) of aqueous solution at each pH | | | | |
|---|---|---|---|---|
| | pH 11 | pH 7 | pH 5 | pH 3 |
| Solution of purified crystals by LR-O method | 921 | 918 | 920 | 914 |

### [Effect of the invention]

By the method of the present invention, the problems of reduced filterability are avoided even when a purified basic amino acid in an alkaline solution is filtered at a neutral to an acidic pH.

## Claims

1. A method for the purification of a basic amino acid wherein an alkaline aqueous solution of an amino acid produced by a fermentation method is filtered through a tight-ultrafiltration membrane.

2. The method according to claim 1, wherein said amino acid is selected from the group consisiting of L-arginine, L-lysine and L-ornithine.
